# FASCICULE DE BREVET EUROPEEN

(11) **EP 2 638 059 B1**
(45) Date de publication et mention de la délivrance du brevet: **11.03.2015**
(21) Numéro de dépôt: 11794201.1
(22) Date de dépôt: 07.11.2011
(51) Int. Cl.: C07K 7/06, C07K 7/08, A61K 38/08, A61K 38/10, A61K 8/64, A61Q 19/08, C07K 5/10

(54) **PEPTIDES ACTIVATEURS DE LA DERMATOPONTINE ET COMPOSITIONS COSMÉTIQUES LES COMPRENANT**
DERMATOPONTIN-AKTIVIERENDE PEPTIDE UND KOSMETISCHE ZUSAMMENSETZUNG DAMIT
DERMATOPONTIN-ACTIVATING PEPTIDES AND COSMETIC COMPOSITIONS INCLUDING SAME

(30) Priorité: 09.11.2010 FR 1004380
(43) Date de publication de la demande: 18.09.2013
(73) Titulaire: ISP Investments Inc., Wilmington, DE 19805 (US)
(72) Inventeur: DAL FARRA, Claude, Kerhonkson New York 12446 (US); DOMLOGE, Nouha, 06560 Valbonne (FR); BOTTO, Jean-Marie, 06560 Valbonne (FR)
(74) Mandataire: Macquet, Christophe
(86) Numéro de dépôt international: PCT/FR2011/000591
(87) Numéro de publication internationale: WO 2012/062977

(56) Documents cités:
- EP-A1- 0 503 939
- WO-A2-00/00214
- WO-A2-2009/012472
- JP-A- 2008 201 777
- US-A1- 2008 071 706
- US-A1- 2010 168 126
- OKAMOTO O ET AL: "Dermatopontin Promotes Epidermal Keratinocyte Adhesian via .alpha.3.beta.1 Integrin and a Proteoglycan Receptor", BIOCHEMISTRY, vol. 49, 24 novembre 2009 (2009-11-24), pages 147-155, XP002632716, cité dans la demande
- ROVERO P ET AL: "Structure-activity Relationship Study of R396, An NK2 Tachykinin Antagonist Selective for the NK2B Receptor Subtype", NEUROPEPTIDES, vol. 23, 1992, pages 143-145, XP002632717,
- KURODA K ET AL: "Dermatopontin Expression is Decreased in Hypertrophic Scar and Systemic Sclerosis Skin Fibroblasts and Is Regulated by Transforming Growth Factor-.beta.1, Interleukin-4, and Matrix Collagen", THE JOURNAL OF INVESTIGATIVE DERMATOLOGY, vol. 112, 1999, pages 706-710, XP002632718,
- MAYOROV A V ET AL: "Solid-phase peptide head-to-side chain cyclodimerization: Discovery of C2-symmetric cyclic lactam hybrid alpha-melanocyte-stimulating hormone (MSH)/agouti-signaling protein (ASIP) analogues with potent activities at the human melanocortin receptors", PEPTIDES, ELSEVIER, AMSTERDAM, vol. 31, no. 10, 1 octobre 2010 (2010-10-01), pages 1894-1905, XP027265275, ISSN: 0196-9781 [extrait le 2010-08-03]
- MENARD R ET AL: "The specificity of the S1' subsite of cysteine proteases", FEBS LETTERS, ELSEVIER, AMSTERDAM, NL, vol. 328, no. 1-2, 9 août 1993 (1993-08-09), pages 107-110, XP025652658, ISSN: 0014-5793, DOI: 10.1016/0014-5793(93)80975-Z [extrait le 1993-08-09]
- JOAKIM E. SWEDBERG ET AL: "Plasmin Substrate Binding Site Cooperativity Guides the Design of Potent Peptide Aldehyde Inhibitors", BIOCHEMISTRY, vol. 50, no. 39, 4 octobre 2011 (2011-10-04), pages 8454-8462, XP55016304, ISSN: 0006-2960, DOI: 10.1021/bi201203y

### SEQUENCE LISTING

<110> ISP Investments INC.
<120> NOUVEAUX PEPTIDES ACTIVATEURS DE LA DERMATOPONTINE ET COMPOSITIONS COSMETIQUES LES COMPRENANT
<130> BV PCT 10-146
<150> FR 1004380
   <151> 2010-11-09
<160> 13
<170> PatentIn version 3.5
<210> 1
   <211> 4
   <212> PRT
   <213> Synthetic peptide
<220>
   <221> MOD_RES
   <222> (4)..(4)
   <223> AMIDATION
<400> 1
   Asp Arg Gln Trp
   1
<210> 2
   <211> 4
   <212> PRT
   <213> Synthetic peptide
<220>
   <221> MOD_RES
   <222> (4)..(4)
   <223> AMIDATION
<400> 2
   Asp Arg Glu Trp
   1
<210> 3
   <211> 6
   <212> PRT
   <213> Synthetic peptide
<220>
   <221> MOD_RES
   <222> (6)..(6)
   <223> AMIDATION
<400> 3
   Asp Arg Gln Trp Asn Tyr
   1 5
<210> 4
   <211> 7
   <212> PRT
   <213> Synthetic peptide
<220>
   <221> MOD_RES
   <222> (7)..(7)
   <223> AMIDATION
<400> 4
   Arg Glu Trp Gln Phe Tyr Cys
   1 5
<210> 5
   <211> 8
   <212> PRT
   <213> Synthetic peptide
<220>
   <221> MOD_RES
   <222> (7)..(7)
   <223> AMIDATION
<220>
   <221> DISULFID
   <222> (7)..(8)
<400> 5
   Arg Glu Trp Gln Phe Tyr Cys Cys
   1 5
<210> 6
   <211> 6
   <212> PRT
   <213> Synthetic peptide
<220>
   <221> MOD_RES
   <222> (6)..(6)
   <223> AMIDATION
<400> 6
   Asp Arg Glu Trp Gln Phe
   1 5
<210> 7
   <211> 8
   <212> PRT
   <213> Synthetic peptide
<220>
   <221> MOD_RES
   <222> (8)..(8)
   <223> AMIDATION
<400> 7
   Asp Arg Gln Trp Asn Tyr Ala Cys
   1 5
<210> 8
   <211> 9
   <212> PRT
   <213> Synthetic peptide
<220>
   <221> MOD_RES
   <222> (8)..(8)
   <223> AMIDATION
<220>
   <221> DISULFID
   <222> (8)..(9)
<400> 8
   Asp Arg Gln Trp Asn Tyr Ala Cys Cys
   1 5
<210> 9
   <211> 8
   <212> PRT
   <213> Synthetic peptide
<220>
   <221> MOD_RES
   <222> (8)..(8)
   <223> AMIDATION
<400> 9
   Asp Arg Glu Trp Gln Phe Tyr Cys
   1 5
<210> 10
   <211> 9
   <212> PRT
   <213> Synthetic peptide
<220>
   <221> MOD_RES
   <222> (8)..(8)
   <223> AMIDATION
<220>
   <221> DISULFID
   <222> (8)..(9)
<400> 10
   Asp Arg Glu Trp Gln Phe Tyr Cys Cys
   1 5
<210> 11
   <211> 6
   <212> PRT
   <213> Synthetic peptide
<220>
   <221> MOD_RES
   <222> (6)..(6)
   <223> AMIDATION
<400> 11
   Asp Arg Gln Trp Lys Phe
   1 5
<210> 12
   <211> 6
   <212> PRT
   <213> Synthetic peptide
<220>
   <221> MOD_RES
   <222> (6)..(6)
   <223> AMIDATION
<400> 12
   Arg Glu Trp Gln Phe Tyr
   1 5
<210> 13
   <211> 6
   <212> PRT
   <213> Synthetic peptide
<400> 13
   Arg Glu Trp Gln Phe Tyr
   1 5

## Description

### Domaine de l'invention

La présente invention se situe dans le domaine des principes actifs cosmétiques et dermato-pharmaceutiques ainsi qu'aux compositions en comportant.

La présente invention a pour objet de fournir de nouvelles molécules utiles pour prévenir ou lutter contre les signes cutanés du vieillissement, et en particulier les rides, le relâchement, la perte de volume et d'élasticité de la peau.

Plus particulièrement, l'invention se rapporte à des peptides activateurs de l'expression de la dermatopontine, à des compositions cosmétiques ou pharmaceutiques comprenant de tels peptides, à l'utilisation de telles compositions pour augmenter l'expression des protéines de la matrice extracellulaire, prévenir la dégradation des fibres de collagène et des fibres élastiques par les UV et enfin, à des méthodes de soin cosmétiques destinées à prévenir et/ou traiter les signes cutanés du vieillissement et du photovieillissement.

### Arrière-plan de l'invention

La peau est un organe de recouvrement composé de plusieurs couches (derme, jonction dermo-épidermique, épiderme) ; Le derme est le tissu de soutien de la peau et se compose d'eau, de fibres d'élastine et de fibres de collagène (70 % des fibres dermiques), enveloppées dans une matrice interstitielle de protéoglycannes. Les fibroblastes sont la principale composante cellulaire du derme et sont à l'origine de la synthèse des fibres de collagène et des fibres d'élastine.

La partie la plus externe est l'épiderme, un épithélium pluristratifié constitué essentiellement de kératinocytes étroitement liés entre eux. L'assisse basale de l'épiderme est composée par une couche de cellules prolifératives, principalement des kératinocytes et des mélanocytes, qui s'ancrent sur la jonction dermo-épidermique (JDE). La JDE est une structure extracellulaire en treillage qui constitue l'interface entre le derme et l'épiderme.

La peau, comme tous les autres organes, est soumise au processus physiologique complexe du vieillissement. On distingue le vieillissement intrinsèque ou chronologique, qui est la conséquence d'une sénescence génétiquement programmée et d'altérations biochimiques dues à des facteurs endogènes. Au niveau de la peau, il se caractérise par un ralentissement de la régénération cellulaire et des matrices extracellulaires, aboutissant à une atrophie dermique et épidermique, une sécheresse, une réduction de l'élasticité, et l'apparition de ridules et de fines rides.

Le vieillissement extrinsèque, quant à lui, est dû à des agressions environnementales que subit l'organisme tout au long de la vie, tels que la pollution, le soleil, les maladies, les habitudes de vie, etc. Il se superpose au vieillissement intrinsèque au niveau des zones chroniquement exposées au soleil ; on parle alors de photovieillissement. Les principales altérations liées au photovieillissement siègent au niveau du derme et comprennent : l'apparition de tâches pigmentaires, une diminution et une fragmentation des fibres de collagène provoquant des rides et l'accumulation de fibres élastiques dystrophiques constituant l'élastose solaire.

De nombreuses voies de recherche ont été explorées pour identifier des agents actifs capables de lutter contre le vieillissement cutané, parmi lesquelles la protection contre les stress environnementaux (soleil, pollutions...), l'activation de la régénération cellulaire, le renforcement de la matrice extracellulaire de collagène et d'élastine. Ces recherches ont abouti à la mise sur le marché de nombreux agents actifs plus ou moins efficaces. De ce fait, il reste toujours d'actualité d'identifier de nouveaux composés capables de prévenir ou de lutter contre le vieillissement cutané. Le problème plus particulièrement ciblé par l'invention est de lutter contre la désorganisation des structures fibrillaires de la matrice extracellulaire cutanée apparaissant lors du vieillissement ou du photovieillissement.

Les inventeurs ont récemment identifié une cible moléculaire intéressante pour remplir cette fonction. Il s'agit de la dermatopontine, une petite protéine acide, riche en tyrosine, présente en abondance dans le derme et plus précisément, autour des fibres de collagène. Elle joue un rôle clé dans la structuration des fibrilles de collagène (Jonathan et al., J. Biol. Chem., 1993, vol. 268, pp. 19826-19832), participe au processus d'adhésion des fibroblastes (Lewandowska et al., J. cell Sci., 1991, vol.99, pp. 657-668), des kératinocytes via l'intégrine α₃β₁ et un récepteur de type protéoglycanne. Ces propriétés font de la dermatopontine un acteur important de la cicatrisation (Akamoto et al., Biochem, 2010, vol. 49, pp. 1.47-155).

Confirmant ces résultats, l'absence de dermatopontine chez des souris génétiquement modifiées abouti à une diminution de l'épaisseur du derme et de sa teneur en collagène, ainsi qu'à une moindre élasticité de la peau (Takeda et al. J. Invest. Dermatol. 2002, vol. 119, pp. 678-683).

Indépendamment de son rôle structural, la dermatopontine peut se lier en un complexe trimérique avec la décorine et le TGF bêta et potentialiser ainsi l'action du TGF bêta 1 (Okamoto et al., Biochem J. 1999 Feb 1;337 (3):537-41).

Le document JP2008201777 divulgue l'utilisation de peptides dérivés de la dermatopontine, en tant qu'agents favorisant l'adhésion cellulaire et la cicatrisation.

Le document US20050065089 divulgue que la dermatopontine native peut être utilisée pour potentialiser l'activation du TGF beta dans le cadre d'un traitement biologique des hernies discales.

US 2010/168126 A1 divulgue l'utilisation d'un dipeptide modifié pour prévenir ou lutter contre les signes cutanés du vieillissement. Ce document décrit que le dipeptide peut activer l'expression de la dermatopontine.

Toutefois, à ce jour, aucun peptide conforme à l'invention n'a été décrit pour activer la dermatopontine dans les cellules de la peau et prévenir ou réparer les signes cutanés du vieillissement et du photovieillissement.

### Exposé de l'invention

Les inventeurs ont mis en évidence que des peptides de formule générale (I) suivante :

R₁-(AA)ₙ-X₁-Arg-X₂-Trp-X₃-X₄-X₅-X₆(R₃)-(AA)ₚ-R₂

étaient de bons agents activateurs de la dermatopontine. En conséquence, ces peptides sont adaptés pour lutter contre le vieillissement et le photovieillissement de la peau.

Les peptides selon l'invention se caractérisent par le fait qu'ils :
- activent l'expression de la dermatopontine,
- augmentent l'expression du collagène de type I, III et de la fibronectine,
- préviennent la dégradation des structures fibrillaires de collagène et des fibres élastiques dans une peau soumise à des rayonnements UV.

On entend par « peptide ou agent actif activateur de la dermatopontine ou capable d'activer la dermatopontine», tout peptide de formule générale (I) capable d'augmenter la quantité de dermatopontine présente dans la cellule, soit en augmentant la synthèse protéique par modulation directe ou indirecte de l'expression génique, soit par d'autres processus biologiques tels que la stabilisation de la protéine ou encore la stabilisation des transcrits d'ARN messager.

On entend par peau, l'ensemble des tissus de recouvrement constituant la peau, les muqueuses et les phanères, y compris les cheveux, les cils et les sourcils.

Ainsi, l'invention a pour objet premier un peptide de formule générale (I)

R₁-(AA)ₙ-X₁-Arg-X₂-Trp-X₃-X₄-X₅-X₆(R₃)-(AA)ₚ-R₂

Dans laquelle
X₁ représente l'acide aspartique ou aucun acide aminé,
X₂ représente la glutamine où l'acide glutamique,
X₃ représente l'asparagine ou la lysine ou la glutamine ou aucun acide aminé,
X₄ représente la phénylalanine ou la tyrosine ou aucun acide aminé,
X₅ représente la tyrosine ou l'alanine ou aucun acide aminé,
X₆ représente la cystéine ou aucun acide aminé,
AA représente un acide aminé quelconque à l'exception de l'arginine, la cystéine, la leucine, la glycine et l'acide glutamique ; et n et p = 0 ou 1 avec n différent de p ;
R₁ représente la fonction amine primaire de l'acide aminé N-terminal, libre ou substituée par un groupement de typé acyle (R-CO-) où le radical R est soit une chaîne alkyle de C₁ à C₃₀ saturée ou insaturée de type acétyle, soit un groupement aromatique de type benzoyle, tosyle, ou benzyloxycarbonyle ;
R₂ représente la fonction carboxyle de l'acide aminé C-terminal possédant soit un groupement hydroxyle libre ou substitué par un groupement choisi parmi une chaîne alkyle de C₁ à C₃₀, soit un groupement -NH₂, -NHY ou -NYY' avec Y et Y' représentant une chaîne alkyle de C₁ à C₄ ;
R₃ représente la fonction thiol de la cystéine en position X₆, soit libre, soit substituée par un groupement méthyle ou acétyle, soit liée de manière covalente par un pont disulfure à une autre cystéine.

Selon une méthode de réalisation de l'invention tout particulièrement préférée, le peptide est de séquence :
(SEQ ID n°1) : Asp-Arg-Gln-Trp-NH₂
(SEQ ID n°2) : Asp-Arg-Glu-Trp-NH₂
(SEQ ID n°3) : Asp-Arg-Gln-Trp-Asn-Tyr-NH₂
(SEQ ID n°4) : Arg-Glu-Trp-Gln-Phe-Tyr-Cys-NH₂
(SEQ ID n°5) : Arg-Glu-Trp-Gln-Phe-Tyr-Cys(Cys)-(NH₂)
(SEQ ID n°6) : Asp-Arg-Glu-Trp-Gln-Phe-NH₂
(SEQ ID n°7) : Asp-Arg-Gln-Trp-Asn-Tyr-Ala-Cys-NH₂
(SEQ ID n°8) : Asp-Arg-Gln-Trp-Asn-Tyr-Ala-Cys(Cys)-(NH₂)
(SEQ ID n°9) : Asp-Arg-Glu-Trp-Gln-Phe-Tyr-Cys-NH₂
(SEQ ID n°10) : Asp-Arg-Glu-Trp-Gln-Phe-Tyr-Cys(Cys)-(NH₂)
(SEQ ID n°11) : Asp-Arg-Gln-Trp-Lys-Phe-NH₂
(SEQ ID n°12) : Arg-Glu-Trp-Gln-Phe-Tyr-NH₂
(SEQ ID n°13) : Arg-Glu-Trp-Gln-Phe-Tyr.

Selon un mode de réalisation particulièrement intéressant, le peptide correspond à la séquence SEQ ID n°5.

Selon un autre mode de réalisation particulièrement intéressant, le peptide correspond à la séquence SEQ ID n°9 ou à la SEQ ID n°10.

Selon un autre mode de réalisation encore plus particulièrement intéressant, le peptide correspond à la séquence SEQ ID n°12 ou à la SEQ ID n°13.

Les acides aminés, constituant le peptide selon l'invention et désignés sous les termes AA, peuvent être sous configuration isomérique L- et D-. De manière préférentielle, les acides aminés sont sous forme L.

Le terme « peptide » désigne un enchaînement de deux ou plusieurs acides aminés liés entre eux par des liaisons peptidiques.

Par «peptide», il faut également entendre le peptide naturel, ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. De préférence, on utilise pour protéger la fonction amine primaire de l'acide aminé N-terminal, une substitution par un groupement R₁ de type acyle (R-CO-) dans lequel le radical R est soit une chaîne alkyle de C₁ à C₃₀ saturée ou insaturée de type acétyle, soit un groupement aromatique de type benzoyle, tosyle, ou benzyloxycarbonyle, encore plus préférentiellement un groupement acétyle. De préférence, on utilise, pour protéger la fonction carboxyle de l'acide aminé C-terminal, une substitution par un groupement R₂ de type chaîne alkyle de C₁ à C₃₀, ou un groupement NH2, NHY ou NYY avec Y représentant une chaîne alkyle de C₁ à C₄, encore plus préférentiellement un groupement NH2.

Le peptide selon l'invention peut être protégé au niveau de l'extrémité N-terminale, C-terminale ou au niveau des deux extrémités.

De façon à inhiber la dimérisation des peptides selon l'invention, la fonction thiol de la cystéine C-terminale peut être substituée par un groupement méthyle, acétyle ou encore une autre cystéine. Dans ce dernier cas, la substitution aboutit à la formation d'un pont disulfure entre les deux résidus cystéines.

Ainsi, l'invention concerne une composition telle que définie précédemment, caractérisée par le fait que le peptide selon l'invention et avantageusement de séquence SEQ ID n°1 à SEQ ID n°13 est sous forme protégée ou non, préférentiellement sous forme protégée au niveau de l'extrémité C-terminale.

Le peptide de formule générale (I) selon l'invention peut être obtenu soit par synthèse chimique classique (en phase solide ou en phase homogène liquide), soit par synthèse enzymatique (Kullman et al. J. Biol. Chem., 1980, vol. 225, p. 8234) à partir d'acides aminés constitutifs.

Le peptide selon l'invention peut être d'origine naturelle ou synthétique. Préférentiellement selon l'invention, le peptide est d'origine synthétique, obtenu par synthèse chimique.

Selon l'invention, l'agent actif peut être un peptide unique ou un mélange de peptides. Le peptide selon l'invention est avantageusement solubilisé dans un ou plusieurs solvants physiologiquement adaptés, tels que l'eau, le glycérol, l'éthanol, le propanediol, le butylène glycol, le dipropylène glycol, les diglycols éthoxylés ou propoxylés, les polyols cycliques ou tout mélange de ces solvants.

On entend par «physiologiquement adapté» que le solvant choisi est approprié pour entrer en contact avec la peau sans provoquer de réactions de toxicité ou d'intolérance.

Le peptide dilué est ensuite stérilisé par filtration stérile.

Après cette étape de dilution, le peptide peut être encapsulé ou inclus dans un vecteur cosmétique ou pharmaceutique tels que les liposomes ou toute autre microcapsule utilisée dans le domaine de la cosmétique ou adsorbé sur des polymères organiques poudreux, des supports minéraux comme les talcs et bentonites, et plus généralement solubilisé dans, ou fixé sur, tout vecteur physiologiquement adapté.

Selon un second objet de l'invention, le peptide de formule générale (I) peut être utilisé en tant que médicament.

De manière particulièrement avantageuse, le peptide de formule générale (I) peut être utilisé en tant qu'agent cicatrisant.

L'invention a encore pour objet une composition pharmaceutique cicatrisante comprenant, dans un milieu physiologiquement adapté, le peptide selon l'invention.

Selon un aspect particulier de l'invention, ledit peptide peut être utilisé pour traiter les symptômes dermatologiques liés à l'âge ou au photovieillissement prématuré, et notamment le retard de cicatrisation, la laxité et l'atrophie dermique, l'élastose solaire et la diminution de la cohésion dermo-épidermique.

Avantageusement, selon cette forme de l'invention, les compositions pharmaceutiques seront appropriées à une application topique ou pourront se présenter sous une forme liquide appropriée pour l'injection sous ou dans la peau, en particulier appropriée aux multi-injections superficielles locales de type mésothérapie.

L'invention a pour troisième objet une composition cosmétique comprenant, dans un milieu physiologiquement adapté, un peptide de formule générale (I), en tant qu'agent actif activateur de la dermatopontine.

Selon un mode de réalisation avantageux de l'invention, l'agent actif selon l'invention est présent dans les compositions de l'invention à une concentration comprise entre 10⁻⁹ M et 10⁻³ M environ, et préférentiellement à une concentration comprise entre 10⁻⁸ M et 10⁻¹⁵M, et encore plus préférentiellement entre 5.10⁻⁵ M et 5.10⁻⁶ M par rapport au poids total de la composition finale.
Cette fourchette de concentrations représente la quantité nécessaire d'agent actif pour obtenir l'effet moléculaire recherché, à savoir, activer l'expression de la dermatopontine, du collagène de type I, III et de la fibronectine.

De manière préférée, la composition selon l'invention se présente sous une forme adaptée à l'application par voie topique comprenant un milieu physiologiquement adapté pour la peau. Par « physiologiquement adapté », on entend des milieux qui conviennent à une utilisation en contact avec la peau ou les phanères humains, sans risque de toxicité, d'incompatibilité, d'instabilité, de réponse allergique et autres effets secondaires.

On entend par « application topique », le fait d'appliquer ou d'étaler l'agent actif selon l'invention, ou une composition le contenant, à la surface de la peau.

Ces compositions pourront notamment se présenter sous forme d'une solution aqueuse, hydro-alcoolique ou huileuse ; d'une émulsion huile-dans-eau, eau-dans-huile ou émulsions multiples ; de gel aqueux ou anhydre ; de colloïde. Ces compositions peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'une crème, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des anti-oxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut, par exemple, citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, siloxanes, PVA ou PVP, et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être présents à des concentrations allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Il est bien entendu que l'agent actif selon l'invention peut être utilisé seul ou bien en association avec d'autres agents actifs.

Avantageusement, les compositions utilisables selon l'invention contiennent, en outre, au moins un autre agent actif destiné à renforcer l'action de l'agent actif selon l'invention, dans le domaine de la prévention et de l'amélioration des signes cutanés du vieillissement, ou encore un autre agent actif permettant d'élargir la gamme de propriétés de la composition considérée.

On peut citer, de manière non limitative, les classes d'ingrédients suivantes : les agents régénérants, anti-âges, antirides, apaisants, anti-radicalaires, anti-glycation, hydratants, antibactériens, antifongiques, kératolytiques, myorelaxants, desquamants, raffermissants, les agents stimulant la synthèse de macromolécules dermiques ou le métabolisme énergétique, les agents modulant la différenciation, la pigmentation ou la dépigmentation cutanée, les agents stimulant la pousse des ongles ou des cheveux, les agents stimulant la microcirculation, les écrans ou filtres solaires ou encore les agents inhibiteurs des métalloprotéinases.

Dans un mode plus particulier de réalisation, la composition selon l'invention comprendra, outre le peptide selon l'invention,
- au moins un composé activateur du cytochrome c et/ou,
- au moins un composé hydratant, tel qu'un composé activateur des aquaporines et/ou,
- au moins un composé activateur des sirtuines et/ou,
- au moins un composé qui augmente l'adhésion cellulaire et/ou,
- au moins un composé qui augmente la production de protéines matricielles telles que le collagène, fibronectine, laminine, glycosaminoglycanes et/ou,
- au moins un composé modulateur de l'activité du protéasome et/ou, ,
- au moins un composé modulateur du rythme circadien et/ou,
- au moins un composé modulateur des protéines HSP et/ou,
- au moins un composé qui augmente l'énergie cellulaire et/ou,
- au moins un composé modulant la pigmentation de la peau et/ou,
- au moins un composé activateur du coenzyme Q10 et/ou,
- au moins un composé améliorant la fonction barrière, tel qu'un composé activateur de la transglutaminase ou de la HMG-CoA réductase et/ou,
- au moins un composé protecteur de la mitochondrie.

Lesdits composés ci-dessus peuvent être d'origine naturelle, comme des hydrolysats peptidiques de plantes, ou encore d'origine synthétique, comme des peptides.

Indépendamment de leurs fonctions, les autres agents actifs associés à l'agent actif selon l'invention dans la composition pourront avoir des structures chimiques très diverses. On peut citer de manière non limitative, les peptides, la vitamine C et ses dérivés, les vitamines du groupe B, la DHEA (dihydroépiandrostérone), les phytostérols, l'acide salicylique et ses dérivés, les rétinoïdes, les flavonoïdes, les amines de sucres, les azoles, les sels métalliques, les extraits peptidiques d'origine végétale ou encore les polymères.

L'invention a pour quatrième objet l'utilisation d'une composition cosmétique, comprenant le peptide de formule générale (I) en tant qu'agent actif, pour renforcer la structure de la jonction dermo-épidermique, stimuler l'expression des composants de la jonction dermo-épidermique, augmenter la densification de la zone supportant la jonction dermo-épidermique, ou encore améliorer l'ancrage des kératinocytes basaux et/ des mélanocytes sur la jonction dermo-épidermique.

L'invention a pour cinquième objet l'utilisation d'une composition cosmétique, comprenant le peptide de formule générale (I) en tant qu'agent actif, pour augmenter l'expression des protéines de la matrice extracellulaire par les fibroblastes de la peau.

Selon un aspect avantageux de l'invention, l'agent actif permet d'augmenter la densité et l'élasticité du derme, et donc la fermeté de la peau, de prévenir ou de lutter contre le relâchement des traits du visage, la perte de volume, l'amincissement de la peau, l'atonie, les ridules, les rides profondes et l'atrophie dermique.

L'invention a pour sixième objet l'utilisation d'une composition cosmétique, comprenant le peptide de formule générale (I) en tant qu'agent actif, pour prévenir la dégradation des fibres de collagène et des fibres élastiques dans la peau soumise aux agressions extérieurs.

On entend par l'expression « agressions extérieures », les agressions que peut produire l'environnement. A titre d'exemple, on peut citer des agressions telles que la pollution, les rayonnements UV, ou encore les produits à caractère irritant. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette.

Il a été mis en évidence que l'agent actif permet de limiter la dégradation et la désorganisation des fibres élastiques et de stimuler la réorganisation des fibres de collagène, consécutives aux rayonnements UV, et plus particulièrement aux rayonnements UVA.

La désorganisation des fibres élastiques désigne l'ensemble des modifications consécutives à des expositions solaires répétées, ainsi que l'accumulation de fibres élastiques dystrophiques typiques de l'élastose solaire. Ces altérations, difficilement réversibles et partiellement responsables de la ptôse ou relâchement cutané lié à l'âge, sont principalement dues aux rayonnements UVA qui pénètrent profondément dans le derme.

Un mode de réalisation particulier de l'invention a pour objet l'utilisation d'une composition cosmétique comprenant le peptide selon l'invention, pour prévenir ou lutter contre les signes inesthétiques liés à l'élastose solaire et/ou à la désorganisation des fibres élastiques causées par les expositions aux rayonnements UV, et plus particulièrement aux rayonnements UVA.

L'invention a pour septième objet l'utilisation d'une composition cosmétique, comprenant le peptide de formule générale (I) en tant qu'agent actif pour augmenter la régénération de l'épiderme et du derme.

On entend par l'expression « régénération de l'épiderme et du derme » que, selon cet aspect avantageux de l'invention, le peptide de l'invention provoque une prolifération et une migration plus importante des kératinocytes et des fibroblastes, ce qui favorise un renouvellement accéléré de l'épiderme et plus généralement une meilleure régénération de la peau.

L'invention a pour huitième objet une méthode de soin cosmétique destiné à prévenir et/ou traiter les signes cutanés du vieillissement et du photovieillissement, caractérisé en ce que l'on applique topiquement sur la peau à traiter une composition selon l'invention.

Par manifestations cutanées du vieillissement, on entend toutes modifications de l'aspect extérieur de la peau et des phanères dues au vieillissement comme, par exemple, l'amincissement de la peau, le relâchement, la perte d'élasticité et l'atonie, les rides profondes et les ridules, la perte de fermeté et de tonicité, et l'atrophie dermique ou toute autre dégradation interne de la peau consécutive à une exposition aux rayonnements UV.

En particulier, l'invention se rapporte à un procédé de traitement cosmétique destiné à protéger la peau contre les agressions dues aux rayonnements UV.

Dans un mode de réalisation particulier, la composition est appliquée avant une exposition au soleil, en tant que soin avant-soleil afin de prévenir la désorganisation des fibres élastiques.

Dans un second mode de réalisation de l'invention, la composition est appliquée après une exposition au soleil, en tant que soin après-soleil afin de réparer les dommages subis par les fibres de collagène et les fibres élastiques.

D'autres avantages et caractéristiques de l'invention apparaîtront mieux à la lecture des exemples donnés à titre illustratif et non limitatif.

### Exemple 1 : Mise en évidence de l'effet activateur des peptides SEQ ID n°5 et SEO ID n°9 sur l'expression de la dermatopontine

Le but de cette étude est de déterminer l'influence du peptide SEQ ID n°5 et du peptide SEQ ID n°9 sur l'expression de la dermatopontine dans les fibroblastes. Pour cela, des marquages spécifiques par immunofluorescence ont été réalisés sur des cultures de fibroblastes humains normaux et sur des peaux maintenues en culture *ex vivo.*

Protocole : Des fibroblastes humains normaux sont cultivés pendant 24 ou 48 heures et traités une fois par jour avec une solution à 10⁻⁶ M de peptide SEQ ID n°5 ou de peptide SEQ ID n°9. Les cellules sont ensuite lavées, fixées au formaldéhyde à 3.7% pendant 10 minutes à température ambiante.

Les échantillons de peau humaine sont mis en culture à l'interface air/liquide. Une solution de peptide SEQ ID n°5 ou de peptide SEQ ID n°9 à 10⁻⁶ M est appliquée topiquement, puis les échantillons sont incubés durant 24 heures ou 48 heures. Ces échantillons de peau sont ensuite fixés avec du formaldéhyde puis inclus dans la paraffine ou fixés avec de l'OCT, par congélation à -20° C. Des coupes de 3 à 4 µm sont alors réalisées (6 µm pour les coupes à froid). L'immunomarquage des échantillons inclus en paraffine est effectué après démasquage des sites spécifiques. L'immunomarquage des échantillons inclus avec de l'OCT est effectué après une fixation à 37°C et de l'acétone froid.

Les cellules fixées ou les coupes sont incubées en présence d'un anticorps polyclonal de lapin spécifique de la dermatopontine (Proteintech group, Réf : 10537-1-AP), puis d'un anticorps secondaire, couplé à un marqueur fluorescent. Après montage dans un milieu *ad hoc,* les cellules sont alors examinées au microscope à Epi-fluorescence (Nikon Eclipse E600 microscope).

Résultats : Dans toutes les conditions testées, on observe une fluorescence plus intense dans les fibroblastes traités par le peptide SEQ ID n°5 ou le peptide SEQ ID n°9 à 10⁻⁶ M que dans les conditions contrôle.
Les peaux humaines montrent une fluorescence plus intense au niveau du derme dans les échantillons traités par le peptide SEQ ID n°5 ou le peptide SEQ ID n°9.

Conclusions : Les peptides SEQ ID n°5 et SEQ ID n°9 stimulent très sensiblement l'expression de la dermatopontine par les fibroblastes du derme.

### Exemple 2 : Etude de l'ultrastructure des cellules cutanées traitées par le peptide SEQ ID n° 5

Le but de cette étude est d'observer les structures subcellulaires des kératinocytes et des fibroblastes traités par le peptide SEQ ID n°5 à 10⁻⁶ M.

### Protocole :

Des KHN ou des fibroblastes sont cultivés en boîte ou en système Transwell, ou des peaux cultivées *ex vivo* sont traités avec une solution à 10⁻⁶ M de peptide SEQ ID n°5 pendant 48 heures (le milieu est changé tous les 24 heures). Les cellules ou les échantillons de peaux sont lavés au PBS, puis sont fixés par la fixation hypertonique de Karnosky (4% Paraformaldéhyde, 5% glutaraldéhyde dans un tampon 0,08M Phosphate), 1 heure à température ambiante, puis 24 heures à 4°C. Les cellules sont détachées du support par grattage, centrifugées 5 minutes à 1000 rpm à 4°C. Le surnageant est éliminé et un tampon de 0,1 M sodium cacodylate est déposé sur le culot. Les cellules sont mélangées à 2 % d'agar puis post-fixées par le tétroxyde d'osmium pendant 1 heure. Les échantillons de peaux sont directement post-fixées par le tétroxyde d'osmium au minimum 1 heure. Les spécimens sont alors déshydratés par passages successifs dans une série d'alcool (de 50 à 100 %). Les cellules ou les échantillons de peaux sont ensuite enrobés dans une résine. La polymérisation s'effectue pendant environ 12 heures à 60°C. Des coupes semi-fines de 0,5 µm sont réalisées à l'ultra-microtome. Les coupes sont déposées sur une lame collée à la chaleur puis colorée au bleu de toluidine. Les lames sont ensuite déshydratées de nouveau et montées dans un milieu adéquat. Après avoir choisi la zone d'étude optimale, le bloc est retaillé à la taille désirée et des coupes ultra-fines sont alors réalisées. Seules les coupes qui ont une couleur « gris-argent » et une taille adéquate sont montées sur la grille de microscopie électronique doublement marquée par de l'uranyl acétate et du citrate de plomb, et sont examinées au microscope à transmission à 60 ou 80 KV.

Résultats : L'étude ultrastructurale montre un enrichissement en organites, et particulièrement en mitochondries dans les kératinocytes traités par le peptide SEQ ID n°5. Dans les cultures en système Transwell®, les contacts intercellulaires entre les kératinocytes apparaissent plus cohésifs et les interdigitations plus marquées par rapport au contrôle non traité. De même, dans les cultures de peau *ex vivo,* des contacts cellulaires plus étroits entre les cellules de la couche basale sont observés.

Dans les fibroblastes en culture traités par le peptide SEQ ID n°5, on observe que les corps cavéolaires, le reticulum endoplasmique rugueux et l'appareil de Golgi, sont sensiblement plus développés que dans les cellules témoins. On observe également une sécrétion plus intense de composants de la matrice extracellulaire que dans les cellules contrôle non traitées.

Conclusions : Dans les kératinocytes en culture, le peptide SEQ ID n°5 à 10⁻⁶ M provoque une stimulation globale de l'activité cellulaire avec, en particulier, une augmentation de la densité des mitochondries.

Dans les fibroblastes, le peptide SEQ ID n°5 induit une augmentation de la synthèse de composants de la matrice extracellulaire.

Dans les cultures de peau *ex vivo,* le peptide SEQ ID n°5 augmente les contacts intercellulaires des kératinocytes basaux.

### Exemple 3: Mise en évidence de l'effet activateur du peptide SEQ ID n°5 sur l'expression de fibronectine

Le but de cette étude est de déterminer l'influence du peptide SEQ ID n°5 sur l'expression de la fibronectine, une protéine de la matrice extracellulaire synthétisée par les fibroblastes. Pour cela, des marquages spécifiques par immunofluorescence ont été réalisés sur culture de fibroblastes et sur des cultures de peaux *ex vivo.*

Protocole : Des fibroblastes humains dermiques en culture sont traités une fois par jour avec une solution à 10⁻⁶ M de peptide SEQ ID n°5 (le milieu contenant l'actif est changé, toutes les 24 heures). Les cellules sont ensuite lavées, fixées au formaldéhyde à 3.7% pendant 10 minutes à température ambiante.

Les échantillons de peau humaine sont mis en culture à l'interface air/liquide. Une solution de peptide SEQ ID n°5 ou de peptide SEQ ID n°9 à 10⁻⁶ M est appliquée topiquement, puis les échantillons sont incubés durant 24 heures ou 48 heures. Ces échantillons de peau sont ensuite fixés avec du formaldéhyde puis inclus dans la paraffine ou fixés avec de l'OCT par congélation à -20° C. Des coupes de 3 à 4 µm sont alors réalisées (6 µm pour les coupes à froid). L'immunomarquage des échantillons inclus en paraffine est effectué après démasquage des sites spécifiques. L'immunomarquage des échantillons inclus avec de l'OCT est effectué après une fixation à 37°C et de l'acétone froid.

Les cellules fixées ou les coupes sont incubées en présence d'un anticorps polyclonal de lapin spécifique de la fibronectine (Sigma, Réf : F-3648), puis d'un anticorps secondaire, couplé à un marqueur fluorescent. Les cellules sont alors examinées au microscope à Epi-fluorescence (Nikon Eclipse E600 microscope).

Résultats : On observe une fluorescence cytoplasmique plus intense dans les fibroblastes traités par le peptide SEQ ID n°5 à 10⁻⁶ M que dans les cellules contrôle.

Dans la peau *ex vivo* la fluorescence se situe principalement dans la partie supérieure du derme papillaire, immédiatement sous la jonction dermo-épidermique.

Conclusions : Le peptide SEQ ID n°5 stimule très sensiblement l'expression de la fibronectine dans les fibroblastes.

Dans la peau humaine, le peptide SEQ ID n°5 stimule la densification de la zone supportant la jonction dermo-épidermique.

### Exemple 4: Mise en évidence de l'effet activateur du peptide SEQ ID n°5 sur l'expression des molécules de la matrice extracellulaire dermique collagène I et collagène III

Le but de cette étude est de déterminer l'influence du peptide SEQ ID n°5 sur l'expression des molécules de la matrice extracellulaire dermique. Pour cela, l'expression des collagènes I et III dans des fibroblastes humains provenant du derme a été étudiée.

Protocole : Des fibroblastes humains normaux sont cultivés pendant 24 ou 48 heures et traités une fois par jour avec une solution à 10⁻⁶ M de peptide SEQ ID n°5 ou de peptide SEQ ID n°9. Les cellules sont ensuite lavées, fixées au formaldéhyde à 3.7% pendant 10 minutes à température ambiante.

Les échantillons de peau humaine sont mis en culture à l'interface air/liquide. Une solution de peptide SEQ ID n°5 ou de peptide SEQ ID n°9 à 10⁻⁶ M est appliquée topiquement, puis les échantillons sont incubés durant 24 heures ou 48 heures. Ces échantillons de peau sont ensuite fixés avec du formaldéhyde puis inclus dans la paraffine ou fixés avec de l'OCT par congélation à -20° C. Des coupes de 3 à 4 µm sont alors réalisées (6 µm pour les coupes à froid). L'immunomarquage des échantillons inclus en paraffine est effectué après démasquage des sites spécifiques. L'immunomarquage des échantillons inclus avec, de l'OCT est effectué après une fixation à 37°C et de l'acétone froid.

Les cellules fixées ou les coupes sont incubées en présence d'un anticorps polyclonal de lapin spécifique du Collagène I (Rockland, Réf : 600-401-103) ou d'un anticorps polyclonal de lapin spécifique du Collagène III (Rockland, Réf: 600-401-105), puis d'un anticorps secondaire couplé à un marqueur fluorescent. Après montage dans un milieu *ad hoc,* les cellules sont alors examinées au microscope à Epi-fluorescence (Nikon Eclipse E600 microscope).

Résultats : On observe une fluorescence plus intense dans les cultures et sur les coupes de peaux traitées par le peptide SEQ ID n°5 à 10⁻⁶ M que dans les conditions contrôle.

Conclusions : Le peptide SEQ ID n°5 à 10⁻⁶ M augmente l'expression du collagène I et du collagène III, deux protéines fibrillaires essentielles de la matrice extracellulaire dermiques.

### Exemple 5 : Mise en évidence de l'effet protecteur du peptide SEQ ID n°5 sur fibres d'élastine soumises à irradiations par les UV

Le but de cette étude est de déterminer l'influence du peptide SEQ ID n°5 sur les fibres élastiques présentes dans le derme. Pour cela, une coloration spécifique des fibres élastiques a été réalisée sur des échantillons de peau cultivés *ex vivo.*

Protocole : Des échantillons de peau humaine sont mis en culture à l'interface air/liquide. Une solution à 10⁻⁶ M de peptide SEQ ID n°5 est appliquée topiquement, puis les échantillons sont incubés durant 48 heures avant d'être irradiés par des UVA à 5 J/cm². Des contrôles non traités et irradiés sont réalisés en parallèle.

Ces échantillons de peau sont ensuite fixés avec du formaldéhyde puis inclus dans la paraffine. Des coupes de 4 µm sont alors réalisées. Ces coupes de peau sont successivement déparaffinées puis réhydratées dans différents bains de xylène et d'alcool. Les fibres d'élastine sont colorées en utilisant un kit de coloration Elastika van Gieson (VWR, Réf: 1.15974). Les coupes de peau colorées sont déshydratées puis montées dans le milieu de montage Eukitt et observées au microscope.

Résultats : Les observations au microscope montrent une moindre dégradation des fibres élastiques, ainsi qu'une meilleure préservation de l'organisation des fibres élastiques dans les échantillons irradiés et traités par le peptide SEQ ID n°5 à 10⁻⁶ M que dans les conditions contrôle.

Conclusion : Le peptide SEQ ID n°5 à 10⁻⁶ M préservent les fibres élastiques des altérations provoquées par les rayonnements UV.

### Exemple 6 : Mise en évidence de l'effet régénérant du peptide SEQ ID n°5

Le but de cette étude est de déterminer l'effet régénérant du peptide SEQ ID n°5 sur les fibroblastes dermiques et les kératinocytes humains normaux (KHN). Pour ce faire, le modèle de cicatrisation *in vitro* Ibidi (Integrated Bio Diagnostics, Munich, Germany) a été utilisé.

Protocole : Ce test consiste à maintenir une zone dépourvue de cellule au milieu d'un tapis cellulaire et à évaluer le temps nécessaire aux cellules disposées de part et d'autre de la « cicatrice » pour migrer ou proliférer et combler la brèche. Les inserts de culture en silicone autocollants sont placés au fond d'un puits d'une plaque de culture. Ces inserts ont la particularité d'être composés de deux chambres distinctes séparées par une membrane imperméable de 500 µ m d'épaisseur. Chacune des deux chambres est ensemencée avec des cellules d'un même type cellulaire et cultivées jusqu'à confluence, L'insert est alors retiré avec une pince stérile créant ainsi une zone acellulaire de 400 µm de large entre les deux tapis cellulaires. Les cellules sont ensuite traitées avec une solution à 10⁻⁶ M ou à 3.10⁻⁶ M de peptide SEQ ID n°5 ajouté au milieu de culture, avec renouvellement du peptide toutes les 24 heures. Des observations en microscopie à contraste de phase (Olympus CK40 microscope X5 connecté à un appareil photo Olympus E-510) ont été réalisées à différents temps (0, 6, 24, 30 et 48 heures) jusqu'à ce que le processus de prolifération et de migration aboutisse au comblement de la brèche.

Résultats : Les observations microscopiques montrent un comblement total de la brèche après 30 heures par les kératinocytes humains traités par le peptide SEQ ID n°5 à 10⁻⁶ M. Dans le même temps, dans les conditions contrôle, la brèche cellulaire n'est pas encore comblée.

Le comblement total de la brèche apparait après 48 heures pour les fibroblastes humains traités par le peptide SEQ ID n°5 à 10⁻⁶ M. Dans le même temps, dans les conditions contrôle, la brèche cellulaire n'est pas encore comblée.

L'effet du peptide est dose-dépendant, puisque le comblement de la brèche est plus rapide lorsque les cellules sont traitées par le SEQ ID n°5 à 3.10⁻⁶ M.

Conclusions : Les fibroblastes et les kératinocytes humains normaux (KHN) ont réinvesti plus rapidement la brèche lorsqu'ils étaient cultivés en présence de peptide SEQ ID n°5, en comparaison des conditions contrôle.

Le peptide SEQ ID n°5 stimule la prolifération et la migration des fibroblastes et des KHN, favorisant ainsi la régénération de la peau.

### Exemple 7: Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 17.89 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n° 5 | | 3.10⁻⁶M |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée à 45°C en augmentant l'agitation. La phase D est ensuite additionnée lorsque la température se situe en dessous de 40°C. Le refroidissement est poursuivi jusqu'à 25°C sous vive agitation.

### 2-Crème remodelante visage :

| ***Noms commerciaux*** | ***Noms INCI*** | **% *massique*** |
|---|---|---|
| ***Phase A*** | | |
| Montanov 68 | Cetearyl (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L-101 | Mineral Oil (and) Lanolin Alcohol | 2,00 |
| Abil 356 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol. | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n°5 | | 1.10⁻⁶ M |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase Ajuste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 3 -Crème protectrice de jour :

| ***Noms commerciaux*** | ***Noms INCI*** | **% *massique*** |
|---|---|---|
| ***Phase A*** | | |
| Emulium Delta | Cetyl alcohol (and) Glyceryl Stearate (and) PEG-75 Stearate (and) Çeteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n°9 | | 2.10⁻⁶ M |

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Peptide de formule générale (I) :
R₁-(AA)ₙ-X₁-Arg-X₂-Trp-X₃-X₄-X₅-X₆(R₃)-(AA)ₚ-R₂
Dans laquelle
X₁ représente l'acide aspartique ou aucun acide aminé,
X₂ représente la glutamine ou l'acide glutamique,
X₃ représente l'asparagine ou la lysine ou la glutamine ou aucun acide aminé,
X₄ représente la phénylalanine ou la tyrosine ou aucun acide aminé,
X₅ représente la tyrosine ou l'alanine ou aucun acide aminé,
X₆ représente la cystéine ou aucun acide aminé,
AA représente un acide aminé quelconque à l'exception de l'arginine, la cystéine, la leucine, la glycine et l'acide glutamique, et n et p = 0 ou 1 avec n différent de p ;
R₁ représente la fonction amine primaire de l'acide aminé N-terminal, libre ou substituée par un groupement de type acyle (R-CO-) où le radical R est soit une chaîne alkyle de C₁ à C₃₀ saturée ou insaturée de type acétyle, soit un groupement aromatique de type benzoyle, tosyle, ou benzyloxycarbonyle ;
R₂ représente la fonction carboxyle de l'acide aminé C-terminal possédant soit un groupement hydroxyle libre ou substitué par un groupement choisi parmi une chaîne alkyle de C₁ à C₃₀, soit un groupement -NH₂, -NHY ou -NYY' avec Y et Y' représentant une chaîne alkyle de C₁ à C₄;
R₃ représente la fonction thiol de la cystéine en position X₆, soit libre, soit substituée par un groupement méthyle ou acétyle, soit liée de manière covalente par un pont disulfure à une autre cystéine.

2. Peptide selon la revendication 1, **caractérisé en ce qu'**il correspond à une des séquences suivantes :
(SEQ ID n°1): Asp-Arg-Gln-Trp-NH₂
(SEQ ID n°2): Asp-Arg-Glu-Trp-NH₂
(SEQ ID n°3): Asp-Arg-Gln-Trp-Asn-Tyr-NH₂
(SEQ ID n°4): Arg-Glu-Trp-Gln-Phe-Tyr-Cys-NH₂
(SEQ ID n°5): Arg-Glu-Trp-Gln-Phe-Tyr-Cys(Cys)-(NH₂)
(SEQ ID n°6): Asp-Arg-Glu-Trp-Gln-Phe-NH₂
(SEQ ID n°7): Asp-Arg-Gln-Trp-Asn-Tyr-Ala-Cys-NH₂
(SEQ ID n°8): Asp-Arg-Gln-Trp-Asn-Tyr-Ala-Cys(Cys)-(NH₂)
(SEQ ID n°9): Asp-Arg-Glu-Trp-Gln-Phe-Tyr-Cys-NH₂
(SEQ ID n°10): Asp-Arg-Glu-Trp-Gln-Phe-Tyr-Cys(Cys)-(NH₂)
(SEQ ID n°11): Asp-Arg-Gln-Trp-Lys-Phe-NH₂
(SEQ ID n°12): Arg-Glu-Trp-Gln-Phe-Tyr-NH₂
(SEQ ID n°13): Arg-Glu-Trp-Gln-Phe-Tyr.

3. Peptide selon l'une des revendications 1 ou 2, **caractérisé en ce qu'**il est solubilisé dans un ou plusieurs solvants physiologiquement adaptés, choisis parmi l'eau, le glycérol, l'éthanol, le propanediol, le butylène glycol, le dipropylène glycol, les diglycols éthoxylés ou propoxylés, les polyols cycliques ou tout mélange de ces solvants.

4. Peptide de formule générale (I) tel que défini dans l'une des revendications 1 à 3, pour son utilisation en tant que médicament.

5. Peptide selon la revendication 4, pour son utilisation en tant qu'agent cicatrisant.

6. Composition cosmétique comprenant dans un milieu physiologiquement adapté, au moins un peptide tel que défini dans l'une des revendications 1 à 3, en tant qu'agent actif activateur de la dermatopontine.

7. Composition selon la revendication 6, **caractérisée en ce que** ledit peptide est présent à une concentration comprise entre 10⁻⁹ M et 10⁻³ M, et préférentiellement entre 10⁻⁸ M et 10⁻⁵ M par rapport au poids total de la composition finale.

8. Composition selon l'une des revendications 6 ou 7, **caractérisée en ce qu'**elle est destinée à une administration par voie topique.

9. Composition selon l'une quelconque des revendications 6 à 8, **caractérisée en ce qu'**elle contient en outre, au moins un autre agent actif.

10. Composition selon la revendication 9, **caractérisée en ce que** l'autre agent actif est choisi parmi les agents régénérants, anti-âges, antirides, apaisants, anti-radicalaires, anti-glycation, hydratants, antibactériens, antifongiques, kératolytiques, myorelaxants, desquamants, raffermissants, les agents stimulant la synthèse de macromolécules dermiques ou le métabolisme énergétique, les agents modulant la différenciation, la pigmentation ou la dépigmentation cutanée, les agents stimulant la pousse des ongles ou des cheveux, les agents stimulant la microcirculation, les écrans ou filtres solaires ou encore les agents inhibiteurs des métallo-protéinases.

11. Utilisation cosmétique d'une composition, comprenant le peptide tel que défini dans l'une des revendications 1 à 3 en tant qu'agent actif, dans un milieu physiologiquement adapté, pour augmenter l'expression des protéines de la matrice extracellulaire par les fibroblastes de la peau.

12. Utilisation cosmétique d'une composition, comprenant le peptide tel que défini dans l'une des revendications 1 à 3 en tant qu'agent actif, dans un milieu physiologiquement adapté, pour augmenter la densité du derme et l'élasticité de la peau, prévenir ou lutter contre le relâchement des traits du visage ou la perte de volume, l'amincissement de la peau, l'atonie, les ridules, les rides profondes et l'atrophie dermique.

13. Utilisation cosmétique d'une composition, comprenant le peptide tel que défini dans l'une des revendications 1 à 3 en tant qu'agent actif, dans un milieu physiologiquement adapté, pour prévenir ou lutter contre la dégradation des fibres de collagène et des fibres élastiques dans la peau soumise à des rayonnements UV.

14. Utilisation cosmétique selon la revendication 13 pour prévenir ou lutter contre les signes inesthétiques liés à l'élastose solaire et/ou à la désorganisation des fibres élastiques causées par les expositions aux rayonnements UV, et plus particulièrement aux rayonnements UVA.

15. Utilisation cosmétique d'une composition, comprenant le peptide tel que défini dans l'une des revendications 1 à 3 en tant qu'agent actif, dans un milieu physiologiquement adapté, pour augmenter la régénération de l'épiderme et du derme.

16. Méthode de soin cosmétique destiné à prévenir et/ou traiter les signes cutanés du vieillissement et du photovieillissement, **caractérisé en ce que** l'on applique topiquement sur la peau à traiter une composition telle que définie dans l'une des revendications 6 à 10.

17. Méthode de soin cosmétique selon la revendication précédente, **caractérisée en ce que** la composition est appliquée avant une exposition au soleil, afin de prévenir la désorganisation des fibres élastiques ou la composition est appliquée après une exposition au soleil, afin de réparer les dommages subis par les fibres de collagène et les fibres élastiques.

## Patentansprüche

1. Peptid mit der allgemeinen Formel (I);
R₁-(AA) ₙ-X₁-Arg-X₂-Trp-X₃-X₄-X₅-X₆ (R₃) - (AA) p-R₂
wobei
X₁ Asparaginsäure oder keine Aminosäure darstellt,
X₂ Glutamin oder Glutaminsäure darstellt,
X₃ Asparagin oder Lysin oder Glutamin oder keine Aminosäure darstellt,
X₄ Phenylalanin oder Tyrosin oder keine Aminosäure darstellt,
X₅ Tyrosin oder Alanin oder keine Aminosäure darstellt,
X₆ Cystein oder keine Aminosäure darstellt,
AA irgendeine Aminosäure darstellt mit Ausnahme von Arginin, Cystein, Leucin, Glycin und Glutaminsäure, und n und p = 0 oder 1, wobei n verschieden von p ist;
R₁ die primäre Aminofunktion der N-terminalen Aminosäure darstellt, frei oder substituiert durch eine Gruppe vom Typ (R-CO-)-Acyl, wobei das Radikal R entweder eine Alkylkette von C₁ bis C₃₀, gesättigt oder ungesättigt vom Typ Acetyl ist, oder eine aromatische Gruppe vom Typ Benzoyl, Tosyl oder Benzyloxycarbonyl;
R₂ die Carboxylfunktion der C-terminalen Aminosäure darstellt, die entweder eine Hydroxylgruppe, frei oder substituiert durch eine Gruppe, ausgewählt aus einer Alkylkette von C₁ bis C₃₀, oder einer -NH₂- -NHY- oder - NYY'-Gruppe aufweist, wobei Y und Y' eine Alkylkette von C₁ bis C₄ darstellen;
R₃ die Thiolfunktion des Cysteins in der Position X₆, entweder frei oder substituiert durch eine Methyl- oder Acetylgruppe oder auf kovalente Weise verbunden durch eine Disulfidbrücke mit einem anderen Cystein, darstellt.

2. Peptid nach Anspruch 1, **dadurch gekennzeichnet, dass** es einer der folgenden Sequenzen entspricht:
(SEQ ID Nr. 1): Asp-Arg-Gln-Trp-NH₂
(SEQ ID Nr. 2): Asp-Arg-Glu-Trp-NH₂
(SEQ ID Nr. 3): Asp-Arg-Gln-Trp-Asn-Tyr-NH₂
(SEQ ID Nr. 4): Arg-Glu-Trp-Gln-Phe-Tyr-Cys-NH₂
(SEQ ID Nr. 5): Arg-Glu-Trp-Gln-Phe-Tyr-Cys(Cys)-(NH₂)
(SEQ ID Nr. 6): Asp-Arg-Glu-Trp-Gln-Phe-NH₂
(SEQ ID Nr. 7): Asp-Arg-Gln-Trp-Asn-Tyr-Ala-Cys-NH₂
(SEQ ID Nr. 8): Asp-Arg-Gln-Trp-Asn-Tyr-Ala-Cys(Cys)-(NH₂)
(SEQ ID Nr. 9): Asp-Arg-Glu-Trp-Gln-Phe-Tyr-Cys-NH₂
(SEQ ID Nr. 10): Asp-Arg-Glu-Trp-Gln-Phe-Tyr-Cys(Cys)-(NH₂)
(SEQ ID Nr. 11): Asp-Arg-Gln-Trp-Lys-Phe-NH₂
(SEQ ID Nr. 12): Arg-Glu-Trp-Gln-Phe-Tyr-NH₂
(SEQ ID Nr. 13): Arg-Glu-Trp-Gln-Phe-Tyr.

3. Peptid nach einem der Ansprüche 1 oder 2, **dadurch gekennzeichnet, dass** es in einem oder in mehreren physiologisch angepassten Lösemitteln ausgewählt aus Wasser, Glycerol, Ethanol, Propandiol, Butylenglycol, Dipropylenglycol, ethoxylierten oder propoxylierten Diglykolen, cyclischen Polyolen oder jeder Mischung dieser Lösemittel solubilisiert ist.

4. Peptid mit der allgemeinen Formel (1), wie in einem der Ansprüche 1 bis 3 definiert, zur Verwendung als Arzneimittel.

5. Peptid nach Anspruch 4 zur Verwendung als Wundheilmittel

6. Kosmetische Zusammensetzung, umfassend in einem physiologisch angepassten Medium mindestens ein Peptid, wie in einem der Ansprüche 1 bis 3 definiert, als Dermatopontin-aktivierender Wirkstoff.

7. Zusammensetzung nach Anspruch 6, **dadurch gekennzeichnet, dass** das Peptid in einer Konzentration zwischen 10⁻⁹ M et 10⁻³ M, und vorzugsweise zwischen 10⁻⁸ M und 10⁻⁵ M mit Bezug auf das Gesamtgewicht der endgültigen Zusammensetzung vorhanden ist.

8. Zusammensetzung nach einem der Ansprüche 6 oder 7, **dadurch gekennzeichnet, dass** sie für eine Verabreichung auf topischem Weg ausgelegt ist.

9. Zusammensetzung nach einem der Ansprüche 6 bis 8, **dadurch gekennzeichnet, dass** sie außerdem mindestens einen weiteren Wirkstoff umfasst.

10. Zusammensetzung nach Anspruch 9, **dadurch gekennzeichnet, dass** der andere Wirkstoff ausgewählt ist aus den Regeneriermitteln, Anti-Aging-Mitteln, Mitteln gegen Falten, lindernd wirkenden Mitteln, Antiradikal-, Anti-Glycations-, Befeuchtungs-, antibakteriellen, antimykotischen, keratolytischen, muskelentspannenden, Hautschuppen-entfernenden, hautstraffenden Mitteln, Mitteln, die die Synthese von Hautmakromolekülen oder den Energiestoffwechsel stimulieren, Mitteln, die die Differenzierung, die Pigmentierung oder die Entpigmentierung der Haut modulieren, Mitteln, die das Wachstum der Nägel oder der Haare stimulieren, Mitteln, die die Mikrozirkulation stimulieren; Sonnenschirmen oder -filtern und auch Mitteln zur Hemmung der Metalloproteinasen.

11. Kosmetische Verwendung einer Zusammensetzung, umfassend das Peptid, wie in einem der Ansprüche 1 bis 3 definiert, als Wirkstoff in einem physiologisch angepassten Medium, um die Expression der Proteine der extrazellulären Matrix durch die Fibroblasten der Haut zu erhöhen.

12. Kosmetische Verwendung einer Zusammensetzung, umfassend das Peptid, wie in einem der Ansprüche 1 bis 3 definiert, als Wirkstoff in einem physiologisch angepassten Medium, um die Dichte der Dermis und die Elastizität der Haut zu erhöhen, um der Entspannung der Gesichtszüge oder dem Verlust von Volumen, der Verdünnung der Haut, der Atonie, den Fältchen, den tiefen Falten und der Hautatrophie vorzubeugen und dagegen zu kämpfen.

13. Kosmetische Verwendung einer Zusammensetzung, umfassend das Peptid, wie in einem der Ansprüche 1 bis 3 definiert, als Wirkstoff in einem physiologisch angepassten Medium, um dem Abbau der Kollagenfasern und der elastischen Fasern in der Haut, die UV-Strahlungen ausgesetzt ist, vorzubeugen und dagegen zu kämpfen.

14. Kosmetische Verwendung nach Anspruch 13, um den unästhetischen Anzeichen, die mit der sonnenbedingten Elastose und/oder der Desorganisation der elastischen Fasern, verursacht durch die Aussetzungen an UV-Strahlungen und insbesondere an UVA-Strahlungen, verbunden sind, vorzubeugen oder dagegen zu kämpfen.

15. Kosmetische Verwendung einer Zusammensetzung, umfassend das Peptid, wie in einem der Ansprüche 1 bis 3 definiert, als Wirkstoff in einem physiologisch angepassten Medium, um die Regenerierung der Epidermis oder der Dermis zu erhöhen.

16. Verfahren zur kosmetischen Pflege, ausgelegt, um den Hautanzeichen der Alterung und der Lichtalterung vorzubeugen und/oder diese zu behandeln **dadurch gekennzeichnet, dass** topisch auf der zu behandelnden Haut eine Zusammensetzung angewendet wird, wie in einem der Ansprüche 6 bis 10 definiert.

17. Verfahren zur kosmetischen Pflege nach dem vorhergehenden Anspruch, **dadurch gekennzeichnet, dass** die Zusammensetzung vor einer Aussetzung an die Sonne angewendet wird, um der Desorganisation der elastischen Fasern vorzubeugen, oder die Zusammensetzung nach einer Aussetzung an die Sonne angewendet wird, um die Beschädigungen zu reparieren, die die Kollagenfasern und die elastischen Fasern erlitten haben.

## Claims

1. Peptide of general formula (I):
R₁- (AA) ₙ-X₁-Arg-X₂-Trp-X₃-X₄-X₅-X₆(R₃)-(AA)ₚ-R₂
Wherein
X₁ represents aspartic acid or no amino acid,
X₂ represents glutamine or glutamic acid,
X₃ represents asparagine or lysine or glutamine or no amino acid,
X₄ represents phenylalanine or tyrosine or no amino acid,
X₅ represents tyrosine or alanine or no amino acid,
X₆ represents cysteine or no amino acid,
AA represents any amino acid with the exception of arginine, cysteine, leucine, glycine and glutamic acid, and n and p = 0 or 1 with n different to p;
R₁ represents the primary amine function of the N-terminal amino acid, free or substituted by a group of the acyl type (R-CO-) wherein the radical R is either a saturated or unsaturated C₁ to C₃₀ alkyl chain of the acetyl type, or an aromatic group of the benzoyl, tosyl, or benzyloxycarbonyl type,
R₂ represents the hydroxyl group of the carboxyl function of the C-terminal amino acid, free or substituted by a group chosen from a C₁ to C₃₀ alkyl chain, or an -NH₂, -NHY or -NYY' group with Y and Y' representing a C₁ to C₄ alkyl chain;
R₃ represents the thiol function of cysteine in the X₆ position, either free or substituted by a methyl or an acetyl group, or bound covalently by a disulphide bond to another cysteine.

2. Peptide according to claim 1, **characterised in that** it corresponds to one of the following sequences:
(SEQ ID n°1): Asp-Arg-Gln-Trp-NH₂
(SEQ ID n°2): Asp-Arg-Glu-Trp-NH₂
(SEQ ID n°3): Asp-Arg-Gln-Trp-Asn-Tyr-NH₂
(SEQ ID n°4): Arg-Glu-Trp-Gln-Phe-Tyr-Cys-NH₂
(SEQ ID n°5): Arg-Glu-Trp-Gln-Phe-Tyr-Cys(Cys)-(NH₂)
(SEQ ID n°6): Asp-Arg-Glu-Trp-Gln-Phe-NH₂
(SEQ ID n°7): Asp-Arg-Gln-Trp-Asn-Tyr-Ala-Cys-NH₂
(SEQ ID n°8): Asp-Arg-Gln-Trp-Asn-Tyr-Ala-Cys(Cys)-(NH₂)
(SEQ ID n°9): Asp-Arg-Glu-Trp-Gln-Phe-Tyr-Cys-NH₂
(SEQ ID n°10): Asp-Arg-Glu-Trp-Gln-Phe-Tyr-Cys(Cys)-(NH₂)
(SEQ ID n°11): Asp-Arg-Gln-Trp-Lys-Phe-NH₂
(SEQ ID n°12): Arg-Glu-Trp-Gln-Phe-Tyr-NH₂
(SEQ ID n°13): Arg-Glu-Trp-Gln-Phe-Tyr.

3. Peptide according to one of claims 1 or 2, **characterised in that** it is solubilised in one or more physiologically suitable solvents, chosen from water, glycerol, ethanol, propanediol, butylene glycol, dipropylene glycol, ethoxylated or propoxylated diglycols, cyclic polyols or any mixture of these solvents.

4. Peptide of general formula (I) as defined in one of claims 1 to 3, for use as a drug.

5. Peptide according to claim 4, for use as a healing agent.

6. Cosmetic composition including, in a physiologically suitable medium, at least one peptide as defined in one of claims 1 to 3, as a dermatopontin-activating agent.

7. Composition according to claim 6, **characterised in that** said peptide is present in a concentration of between 10⁻⁹ M and 10⁻³ M, and preferably 10⁻⁸ M and 10⁻⁵ M with respect to the total weight of the final composition.

8. Composition according to one of claims 6 or 7, **characterised in that** it is intended for topical administration.

9. Composition according to any one of claims 6 to 8, **characterised in that** it also contains at least one other active agent.

10. Composition according to claim 9, **characterised in that** the other active agent is chosen from the regenerating, anti-aging, anti-wrinkle, soothing, anti-free radical, anti-glycation, hydrating, antibacterial, antifungal, keratolytic, muscle relaxing, exfoliating, and toning agents, agents stimulating the synthesis of dermal macromolecules or energy metabolism, agents modulating cutaneous differentiation, pigmentation or depigmentation, agents stimulating nail or hair growth, agents stimulating microcirculation, sunscreens or metalloproteinase-inhibiting agents.

11. Cosmetic use of a composition, including the peptide as defined in one of claims 1 to 3 as an active agent, in a physiologically suitable medium, to increase the expression of proteins of the extracellular matrix by the fibroblasts of the skin.

12. Cosmetic use of a composition, including the peptide as defined in one of claims 1 to 3 as an active agent, in a physiologically suitable medium, to increase the density of the dermis and the elasticity of the skin, prevent or fight sagging of the lines of the face or loss of volume, thinning of the skin, atony, fine lines, deep wrinkles and dermal atrophy.

13. Cosmetic use of a composition, including the peptide as defined in one of claims 1 to 3 as an active agent, in a physiologically suitable medium, to prevent or fight degradation of collagen and elastic fibres in the skin subjected to UV radiation.

14. Cosmetic use according to claim 13 to prevent or fight visually unappealing signs related to solar elastosis and/or disorganisation of the elastic fibres caused by exposure to UV radiation, and more specifically to UVA radiation.

15. Cosmetic use of a composition, including the peptide as defined in one of claims 1 to 3 as an active agent, in a physiologically suitable medium, to increase regeneration of the epidermis and the dermis.

16. Cosmetic care method intended to prevent and/or treat the skin signs of aging and photoageing, **characterised in that** a composition as defined in one of claims 6 to 10 is topically applied to the skin to be treated.

17. Cosmetic care method according to the previous claim, **characterised in that** the composition is applied before exposure to the sun, to prevent disorganisation of the elastic fibres, or the composition is applied after exposure to the sun, to repair damage suffered by collagen and elastic fibres.
